# FASCICULE DE BREVET EUROPEEN

(11) **EP 3 307 195 B1**
(45) Date de publication et mention de la délivrance du brevet: **24.04.2024**
(21) Numéro de dépôt: 16739209.1
(22) Date de dépôt: 10.06.2016
(51) Int. Cl.: A61B 34/30, A61B 34/35, B25J 9/00, B25J 9/16

(54) **CHAINE DE TRANSMISSION DE MOUVEMENT ENTRE DES ACTIONNEURS ET UN SOCLE D'ORGANE D'ENTRAINEMENT D'UN ELEMENT MOBILE**
KETTE ZUR ÜBERTRAGUNG EINER BEWEGUNG ZWISCHEN AKTUATOREN UND DER BASIS EINES ELEMENTS ZUM ANTREIBEN EINES BEWEGLICHEN ELEMENTS
CHAIN FOR TRANSMITTING MOVEMENT BETWEEN ACTUATORS AND THE BASE OF A MEMBER FOR DRIVING A MOVABLE ELEMENT

(30) Priorité: 12.06.2015 FR 1555377
(43) Date de publication de la demande: 18.04.2018
(73) Titulaire: Robocath, 76000 Rouen (FR)
(72) Inventeur: DEBOEUF, Sébastien, 76240 Bonsecours (FR); DESTREBECQ, Fabien, 27520 Bourgtheroulde (FR); FOURNIER, Bruno, 41100 Saint Ouen (FR); BENCTEUX, Philippe, 76160 St Martin du Vivier (FR)
(74) Mandataire: Plasseraud IP
(86) Numéro de dépôt international: PCT/FR2016/051400
(87) Numéro de publication internationale: WO 2016/198800

(56) Documents cités:
- WO-A1-2008/115151
- WO-A1-2014/135813
- US-A1- 2009 082 722
- US-A1- 2014 277 333

## Description

La présente invention est relative aux chaînes de transmission de mouvement entre plusieurs actionneurs et un organe d'entraînement d'un élément mobile. L'élément mobile peut notamment être un cathéter ou un guide de cathéter, mais pas nécessairement.

L'insertion manuelle d'un cathéter ou d'un guide dans un patient est un acte chirurgical relativement classique. Toutefois, cet acte étant monitoré sous rayons X, le chirurgien en charge de cet acte est soumis à une irradiation conséquente s'il réalise une telle opération sur de nombreux patients.

Afin de réduire les risques pour le chirurgien, on tente de robotiser une telle insertion. Cette robotisation est complexe, car la préhension du cathéter est difficile. Celui-ci baigne en effet dans du liquide de conservation et doit rester stérile. Par ailleurs, on veut pouvoir commander des mouvements de translation et de rotation alternatifs et/ou simultanés du cathéter. La fiabilité de ces systèmes robotisés est un critère déterminant.

Dans des modules robotisés de l'art antérieur, que ce soit dans le domaine médical des cathéters ou dans d'autres domaines, les actionneurs, qui transmettent leur mouvement à l'organe d'entraînement, le transmettent par l'intermédiaire d'interfaces respectives entre les actionneurs respectifs et le socle de l'organe d'entraînement.

Or, dans ces modules robotisés de l'art antérieur, les interfaces sont situées à l'extérieur du socle de l'organe d'entraînement ou en région périphérique du socle de l'organe d'entraînement.

La structure d'implémentation de ces interfaces entre actionneurs et socle d'organe d'entraînement est alors relativement simple.

L'invention a toutefois détecté un problème de fiabilité de la transmission du mouvement entre actionneurs et socle d'organe d'entraînement dans ce cas-là.

En effet, l'invention a mis en évidence que ce problème de fiabilité vient du caractère décentré de la position des interfaces, entraînant alors une transmission déséquilibrée de l'effort.

Par ailleurs, chaque actionneur ne supporte que l'effort dans sa direction, il n'a pas à porter un ou plusieurs autre actionneurs dans une ou plusieurs autres directions, comme ce pourrait être le cas dans des systèmes existants : l'encombrement et le poids en sont nettement réduits.

C'est pourquoi, l'invention propose de disposer les interfaces de manière à ce que leur intersection soit située en région centrale du socle de l'organe d'entraînement, voire préférentiellement au centre de gravité du socle de l'organe d'entraînement, permettant alors une transmission équilibrée de l'effort, entraînant une transmission fiable du mouvement entre actionneurs d'une part et socle d'organe d'entraînement d'autre part.

Cela implique de faire entrer les interfaces à l'intérieur du socle de l'organe d'entraînement, ce qui rend la structure relativement plus complexe, mais nettement plus fiable au niveau de la qualité de transmission du mouvement entre actionneurs d'une part et socle d'organe d'entraînement d'autre part.

Le socle de l'organe d'entraînement est solidaire de l'organe d'entraînement et fixe par rapport à l'organe d'entraînement.

Le document US 2009/082722 décrit une chaîne de transmission de mouvement selon le préambule de la revendication 1.

Le document WO 2008/115151 décrit une chaîne de transmission de mouvement comprenant trois actionneurs de mouvements respectivement dans les directions X, Y et Z.

A cet effet, selon l'invention, on prévoit une chaîne de transmission de mouvement comprenant :
- un socle d'organe d'entraînement d'un élément mobile,
- trois actionneurs pilotant le socle de l'organe d'entraînement respectivement selon trois directions de translation distinctes entre elles, par l'intermédiaire de trois interfaces respectives avec le socle de l'organe d'entraînement,
caractérisée en ce que l'intersection des surfaces moyennes des trois interfaces est localisée en région centrale du socle de l'organe d'entraînement.

A cet effet, selon l'invention, on prévoit aussi une chaîne de transmission de mouvement comprenant :
- un socle d'un organe d'entraînement d'un élément mobile,
- trois actionneurs pilotant le socle de l'organe d'entraînement respectivement selon trois directions de translation distinctes entre elles, par l'intermédiaire de trois interfaces respectives avec le socle de l'organe d'entraînement,

caractérisée en ce que les trois interfaces sont sensiblement planes,
en ce que ces trois interfaces sont orthogonales les unes aux autres,
et en ce que ces trois interfaces sont imbriquées les unes dans les autres.

Dans des modes de réalisation préférés de l'invention, on peut éventuellement avoir recours en outre à l'une et/ou à l'autre des dispositions suivantes.

Préférentiellement, les trois directions de translation sont orthogonales les unes aux autres.

De préférence, les trois interfaces sont sensiblement planes, ces trois interfaces sont orthogonales les unes aux autres, et ces trois interfaces sont imbriquées les unes dans les autres. Ainsi, les trois interfaces peuvent être concentrées de manière relativement simple et vraiment efficace en région centrale du socle de l'organe d'entraînement.

De préférence, les trois interfaces sont des plaques de pression transmettant les poussées respectives des trois actionneurs. Ces plaques de forme plane permettent une transmission efficace des poussées des actionneurs pour un encombrement global relativement réduit.

De préférence, la première plaque comprend deux ouvertures orthogonales entre elles qui sont respectivement traversées par la deuxième plaque et par la troisième plaque, la deuxième plaque comprend une ouverture qui est traversée par la troisième plaque, l'ouverture de la deuxième plaque étant orthogonale aux deux ouvertures de la première plaque, la troisième plaque n'est traversée ni par la première plaque ni par la deuxième plaque. Cette façon d'imbriquer les plaques entre elles est relativement simple structurellement tout en restant efficace.

De préférence, chacune des ouvertures autorise un débattement de la plaque qui la traverse, ce débattement correspondant à la course de l'actionneur de la plaque qui traverse ladite ouverture, ce débattement étant supérieur à l'épaisseur de la plaque qui traverse ladite ouverture. En effet, si l'un des actionneurs bouge, le socle de l'organe d'entraînement ne doit bouger que dans la direction correspondant à cet actionneur ayant bougé et non pas dans les deux directions correspondant aux actionneurs restés immobiles. Pour cela, la présence de ces débattements permet de désolidariser les unes des autres les transmissions d'effort en provenance de chacun des différents actionneurs.

De préférence, chaque plaque est mobile en translation selon une direction parallèle à la droite constituée par l'intersection des deux autres plaques. Ainsi, la transmission des efforts deux à deux orthogonaux entre les actionneurs, est maintenue aisément.

De préférence, chaque plaque est reliée à son actionneur par deux barres symétriques par rapport à l'axe de poussée dudit actionneur, de préférence par quatre barres symétriques par rapport à l'axe de poussée dudit actionneur. Ainsi, la transmission d'effort en provenance de l'actionneur est bien répartie sur la plaque correspondante.

De préférence, le socle de l'organe d'entraînement est solidarisé de manière fixe à chacune des interfaces de manière à ce que le déplacement de l'une des interfaces entraîne automatiquement le même déplacement du socle de l'organe d'entraînement. Ainsi, la transmission d'effort entre d'une part les interfaces et d'autre part le socle d'organe d'entraînement est plus directe.

De préférence, le socle de l'organe d'entraînement est un cube à l'intérieur duquel sont situées les trois interfaces. Ainsi, le volume global du socle de l'organe d'entraînement est relativement réduit, tandis que les interfaces sont toutefois totalement incluses dans le socle de l'organe d'entraînement. La compacité globale s'en trouve par conséquent améliorée.

De préférence, le socle de l'organe d'entraînement est un cube résultant de l'assemblage de huit cubes plus petits assemblés autour des interfaces. Ces huit petits cubes représentent le nombre minimal de sous parties du cube constituant le socle de l'organe d'entraînement, de manière à pouvoir assembler ce cube autour de l'ensemble des trois interfaces imbriquées entre elles.

De préférence, chaque plaque est coincée entre quatre cubes plus petits d'un côté et quatre cubes plus petits de l'autre côté. Le socle d'organe d'entraînement est ainsi complètement symétrique et équilibré.

De préférence, la région centrale est le centre de gravité du socle de l'organe d'entraînement. La transmission des efforts entre actionneurs et socle d'organe d'entraînement est ainsi parfaitement équilibrée grâce au caractère alors parfaitement centré des interfaces par rapport au socle de l'organe d'entraînement.

De préférence, la chaîne de transmission de mouvement comprend un élément mobile entraîné par l'organe d'entraînement.

Préférentiellement, les matériaux utilisés sont des matériaux à faible voire à très faible friction, pour permettre aux interfaces imbriquées les unes dans les autres de coulisser aisément.

Dans une application préférentielle mais non exclusive, l'élément mobile est un cathéter ou un guide de cathéter, l'organe d'entraînement est un organe de serrage d'un cathéter ou d'un guide de cathéter.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 est une vue schématique de côté d'une installation d'artériographie robotisée,
- la figure 2 est une vue schématique en perspective d'une portion d'un module d'entraînement en configuration libre,
- la figure 3 est une vue en perspective d'un exemple de réalisation de système d'actionnement,
- la figure 4 est une vue en perspective d'un exemple de réalisation de l'intersection et de l'imbrication des interfaces entre actionneurs d'une part et socle d'organe d'entraînement d'autre part,
- la figure 5 est une vue en perspective d'un exemple de réalisation du socle d'organe d'entraînement,
- la figure 6 est une vue en perspective d'un exemple de réalisation d'assemblage entre d'une part les interfaces et d'autre part le socle d'organe d'entraînement,
- la figure 7 est une autre vue en perspective d'un exemple de réalisation d'assemblage entre d'une part les interfaces et d'autre part le socle d'organe d'entraînement.

Sur les différentes figures, les mêmes références désignent des éléments identiques ou similaires.

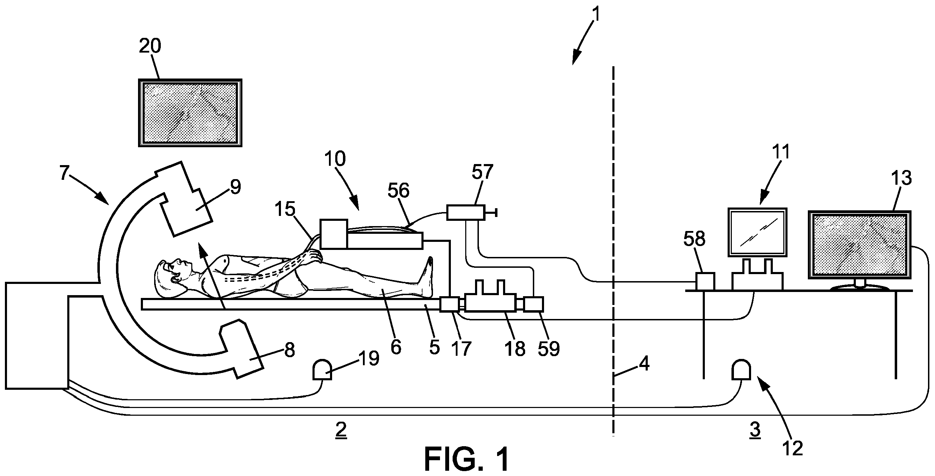

La figure 1 représente schématiquement une installation d'artériographie 1. L'installation d'artériographie 1 est divisée en deux endroits distincts, une salle d'opérations 2 et une salle de commande 3. La salle de commande 3 peut être proche de la salle d'opération 2, séparée de celle-ci par une simple paroi 4, opaque aux rayons X, ou distante. Les matériels de la salle d'opération 2 et de la salle de commande 3 sont reliés entre eux de manière fonctionnelle, par voie filaire, sans fil ou réseau, ....

La salle d'opérations 2 comprend une table d'opérations 5 recevant un patient 6. La salle d'opérations 2 peut également comprendre un imageur médical 7, notamment un imageur par rayons X, comprenant une source 8 et un détecteur 9 disposés de part et d'autre du patient, éventuellement mobiles par rapport au patient.

L'installation d'artériographie 1 comprend un robot 10 disposé dans la salle d'opérations 2.

L'installation d'artériographie 1 comprend un poste de commande 11 disposé dans la salle de commande 3. Le poste de commande 11 est adapté pour commander à distance le robot 10. L'installation d'artériographie 1 peut également comprendre, disposée dans la salle de commande 3, une ou plusieurs commandes distantes 12 de l'imageur 7, communiquant avec l'imageur 7 pour commander celui-ci à distance. L'installation d'artériographie 1 peut également comprendre, disposé dans la salle de commande 3, un écran 13, communiquant avec l'imageur 7, pour visualiser en temps réel dans la salle de commande 3 les images acquises par l'imageur 7.

Le robot 10 peut comprendre un récipient adapté pour contenir un organe médical souple allongé 15 à introduire dans le corps d'un patient. A titre d'organe médical souple allongé 15, il peut par exemple s'agir d'un organe à introduire dans un canal d'un patient, et à déplacer dans ce canal, notamment une artère ou une veine d'un patient, à travers un désilet ménageant une ouverture d'accès dans le patient. L'organe médical souple allongé peut être notamment un cathéter. En variante, l'organe médical souple allongé peut être un guide pour cathéter. Un guide est généralement de diamètre transversal inférieur à celui du cathéter, qui est généralement creux sur une portion proche du patient, voire sur la totalité de sa longueur, de sorte que le guide puisse se déplacer à l'intérieur de celui-ci, notamment à l'intérieur du corps du patient. Le guide peut également comporter une extrémité recourbée.

Le robot 10 peut comprendre un module d'entraînement de l'organe médical souple allongé 15. Le module d'entraînement est commandable à partir du poste de commande 11 pour entraîner l'organe médical souple allongé par rapport au patient selon au moins un degré de liberté, comme ce sera décrit en détails par la suite. Le module d'entraînement peut comprendre un boîtier de communication 17 servant à l'interfaçage avec le poste de commande 11. Au besoin, le robot 10 peut comprendre un boîtier de commande 18 en local, destiné à commander le robot depuis la salle d'opérations 2 si nécessaire.

On notera d'ailleurs que toutes les commandes et les retours disponibles dans la salle de commande 3 peuvent être également disponibles dans la salle d'opérations 2 en vue d'une opération en local, comme par exemple une commande 19 de l'imageur et un écran 20 permettant de visualiser les images acquises par l'imageur 7.

L'organe médical souple allongé 15 creux peut être raccordé à un raccord 56 permettant l'injection d'un produit de contraste facilitant l'imagerie à l'intérieur de l'organe médical souple allongé. L'installation d'artériographie peut comprendre un injecteur 57 de produit de contraste raccordé au raccord 56, commandable par une commande 58 disposée dans la salle de commande 3. Une commande 59 de l'injecteur de produit de contraste peut également être présente en local dans la salle d'opérations 2.

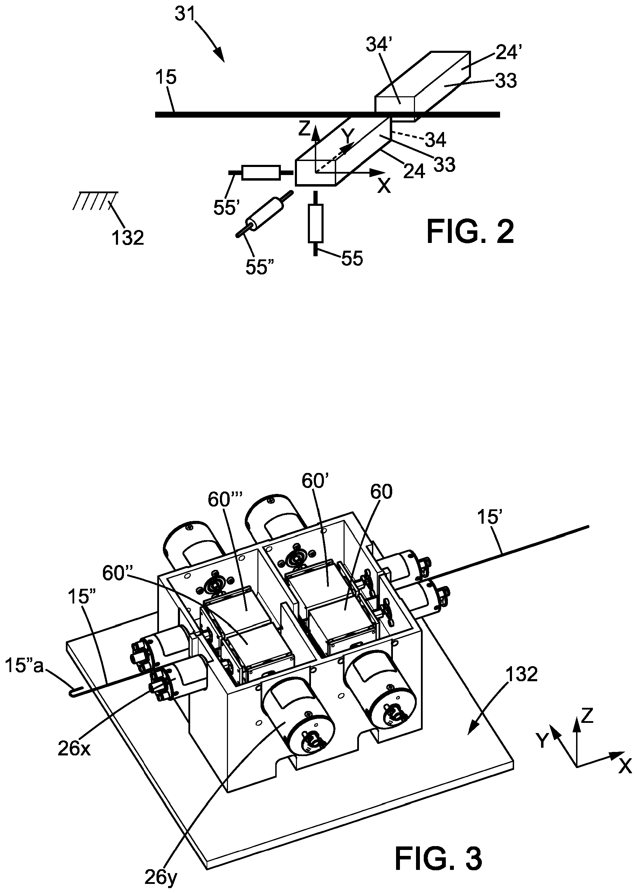

Sur la figure 2, on a représenté un module d'entraînement 31 selon un premier mode de réalisation. Ce module d'entraînement 31 est adapté pour entraîner un organe médical souple allongé 15 s'étendant selon une direction longitudinale X. On notera que la direction longitudinale X au niveau du module d'entraînement 31 n'est pas forcément la même que celle de l'organe médical souple allongé 15 au niveau de son extrémité mais qu'une translation et/ou une rotation de l'organe médical souple allongé 15 selon/autour de la direction longitudinale X au niveau du module d'entraînement 31 entraînera une translation et/ou une rotation de l'organe médical souple allongé 15, respectivement, selon/autour sa direction longitudinale au niveau de son extrémité.

Le module d'entraînement 31 comprend une base 132 et au moins un organe d'entraînement 24 monté mobile par rapport à la base 132. L'organe d'entraînement 24 est par exemple monté mobile par rapport à la base 132.

Dans l'exemple présenté, le module d'entraînement 31 comprend en outre un deuxième organe d'entraînement 24'. L'organe d'entraînement 24, aussi appelé par la suite premier organe d'entraînement, et le deuxième organe d'entraînement 24' forment ensemble une paire d'organes d'entraînement 33. Une paire d'organes d'entraînement 33 comprend deux organes d'entraînement qui coopèrent ensemble pour générer un mouvement de l'organe médical souple allongé 15 par rapport à la base 132. Dans l'exemple présenté, le deuxième organe d'entraînement 24' est monté mobile par rapport à la base 132. Le deuxième organe d'entraînement 24' est par exemple monté mobile par rapport à la base 132.

Le premier organe d'entraînement 24 et le deuxième organe d'entraînement 24' sont appariés pour des mouvements simultanés. Par exemple, les premier et deuxième organes d'entraînement 24, 24' peuvent être commandés individuellement indépendamment l'un de l'autre, mais selon des commandes respectives synchronisées. En variante, on peut prévoir une commande commune qui va être distribuée à l'un et à l'autre des premier et deuxième organes d'entraînement 24, 24' par une liaison mécanique ou électronique entre leurs systèmes de commande.

Chaque organe d'entraînement 24, 24' comporte une surface d'entraînement 34, 34' respectivement. L'organe médical souple allongé 15 est disposé entre les surfaces d'entraînement 34, 34' des organes d'entraînement 24, 24' d'une même paire. Pour fixer les idées, les surfaces d'entraînement 34, 34' sont espacées l'une de l'autre selon la direction Y.

La paire d'organes d'entraînement 24, 24' peut être placée dans une configuration libre, représentée sur la figure 2, dans laquelle la surface d'entraînement 34, 34' des organes d'entraînement 24, 24' de la paire d'organes d'entraînement 33 n'est pas en prise avec l'organe médical souple allongé 15.

La paire d'organes d'entraînement 33 est plaçable dans une configuration d'entraînement dans laquelle les surfaces d'entraînement 34, 34' des organes d'entraînement de la paire d'organes d'entraînement sont en prise avec l'organe médical souple allongé 15 à entraîner. La force appliquée par un organe d'entraînement sur l'organe médical souple allongé dans cette configuration est par exemple de l'ordre de quelques Newtons (5-30 N par exemple). Les moyens de rappel, décrits plus haut, sont par exemple agencés pour ramener la paire d'organes d'entraînement en configuration libre, ce qui permet de fournir une fonction sécuritaire, par exemple en cas de rupture d'alimentation électrique.

Pour placer la paire d'organes d'entraînement 33 alternativement dans les configurations libre et d'entraînement, on peut commander un déplacement relatif l'un vers l'autre des deux organes d'entraînement 24, 24'. Ce déplacement peut par exemple être le déplacement d'un organe d'entraînement 24 par rapport à la base, l'autre restant fixe. En variante, les deux organes d'entraînement 24, 24' peuvent tous deux se déplacer l'un vers l'autre par rapport à la base.

Dans l'exemple, on prévoit un déplacement selon la direction Y.

Dans le mode de réalisation présenté, les deux organes d'entraînement 24, 24' sont mobiles par rapport à la base selon un degré de liberté. Ce degré de liberté diffère de celui permettant le placement alternatif des organes d'entraînement entre les positions libre et d'entraînement. On prévoit notamment que les organes d'entraînement 24, 24' sont mobiles par rapport à la base selon un degré de liberté dans leur configuration d'entraînement. Ainsi, le déplacement des organes d'entraînement selon un degré de liberté dans leur configuration d'entraînement génère un déplacement de l'organe médical souple allongé par rapport à la base 132.

Un exemple de mise en œuvre pratique d'un tel système est présenté ci-après en relation avec la figure 3. Cet exemple de réalisation est fourni de manière illustrative uniquement d'un exemple de réalisation concret d'un système d'actionnement.

La figure 3 comprend une base 132 fixe commune à quatre systèmes d'actionnement. Chaque système d'actionnement commande le déplacement d'un organe d'entraînement respectif, non représenté, mais solidaire d'un cube respectif 60, 60', 60'', 60'''. Les cubes 60, 60', 60'', 60''' correspondent respectivement à des organes d'entraînement non représentés sur la figure. Ces cubes 60, 60', 60'', 60''' sont les socles des organes d'entraînement correspondants.

On utilise la référence 15 pour désigner alternativement le guide 15", le cathéter 15', ou de manière générale un organe médical souple allongé à introduire dans le corps d'un patient. Il peut par exemple s'agir d'un cathéter interventionnel. Un tel cathéter interventionnel peut être de diamètre inférieur au cathéter, de manière à être guidé à l'intérieur de celui-ci, coaxialement à l'intérieur du patient, et être creux de manière à être guidé sur le guide à l'intérieur du patient.

Le guide 15'' présente une extrémité avant 15"a légèrement courbée par rapport à l'axe longitudinal principal du guide, et débouchant par l'extrémité avant du cathéter 15'. Le cathéter 15' peut être soumis à deux mouvements distincts :
- Une translation selon son axe longitudinal,
- Une rotation autour de son axe longitudinal.

Ces mouvements peuvent être générés dans un sens ou dans l'autre.

Le cas échéant, le cathéter 15' peut être soumis à un mouvement combiné des deux mouvements simples décrits ci-dessus.

Le cas échéant, le cathéter 15' peut être soumis à deux mouvements combinés des deux mouvements simples décrits ci-dessus, selon des combinaisons différentes.

Ce qui a été décrit ci-dessus concernant le cathéter s'applique également au guide.

Dans certains cas, le cathéter est lui-même pourvu d'une extrémité courbe, soit pour permettre la navigation sur le même principe qu'un guide, soit pour faciliter le positionnement dans une zone anatomique présentant une courbure particulière.

Par la suite, seule l'opération d'un cube sera décrite. On fait par exemple référence au cube 60'' . Le cube 60" est associé à trois actionneurs 26x, 26y, 26z (ce dernier non visible, similaire en tout point aux actionneurs 26x et 26y, et situé sous la base 132. L'actionneur 26y est utilisé pour déplacer le cube 60" selon la direction Y, tout en autorisant un déplacement du cube 60" à la fois selon les directions X et Z par rapport à l'actionneur 26y dans une certaine plage de déplacement.

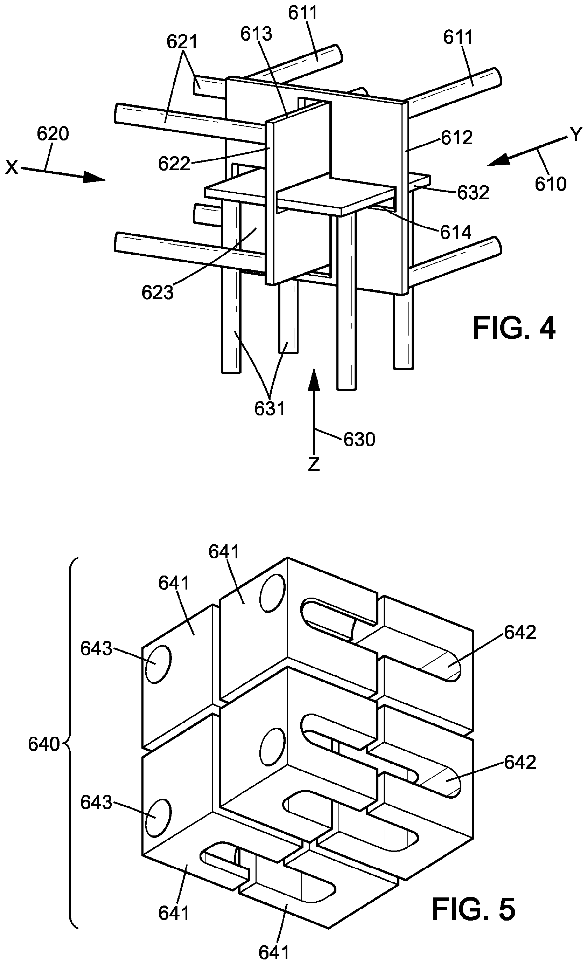

La figure 4 est une vue en perspective d'un exemple de réalisation de l'intersection et de l'imbrication des interfaces entre actionneurs d'une part et socle d'organe d'entraînement d'autre part.

Trois actionneurs 610, 620, 630 exercent un effort selon trois directions Y, X, Z, orthogonales entre elles.

L'actionneur 610 exerce son effort selon la direction Y par l'intermédiaire de 4 barres 611 poussant aux 4 coins d'une première plaque 612 de pression qui constitue l'interface entre l'actionneur 610 et le socle d'organe d'entraînement. La première plaque 612 comprend une première ouverture 613 traversée par une deuxième plaque 622 constituant l'interface entre l'actionneur 620 et le socle d'organe d'entraînement. Cette première ouverture 613 comprend un débattement dans la direction X de manière à permettre la course de l'actionneur 620 et de la deuxième plaque 622 associée selon la direction X sans déplacer la première plaque 612. La première plaque 612 comprend une deuxième ouverture 614 traversée par une troisième plaque 632 constituant l'interface entre l'actionneur 630 et le socle d'organe d'entraînement. Cette deuxième ouverture 614 comprend un débattement dans la direction Z de manière à permettre la course de l'actionneur 630 et de la troisième plaque 632 associée selon la direction Z sans déplacer la première plaque 612.

L'actionneur 620 exerce son effort selon la direction X par l'intermédiaire de 4 barres 621 poussant aux 4 coins d'une deuxième plaque 622 de pression qui constitue l'interface entre l'actionneur 620 et le socle d'organe d'entraînement. La deuxième plaque 622 comprend une troisième ouverture 623 traversée par une troisième plaque 632 constituant l'interface entre l'actionneur 630 et le socle d'organe d'entraînement. Cette troisième ouverture 623 comprend un débattement dans la direction Z de manière à permettre la course de l'actionneur 630 et de la troisième plaque 632 associée selon la direction Z sans déplacer la deuxième plaque 622.

L'actionneur 630 exerce son effort selon la direction Z par l'intermédiaire de 4 barres 631 poussant aux 4 coins d'une deuxième plaque 632 de pression qui constitue l'interface entre l'actionneur 630 et le socle d'organe d'entraînement. La troisième plaque 632 ne comprend aucune ouverture.

La figure 5 est une vue en perspective d'un exemple de réalisation du socle d'organe d'entraînement.

Le socle de l'organe d'entraînement comprend un cube 640 qui pourrait être l'un quelconque des cubes 60, 60', 60'' ou 60''' de la figure 3. Ce cube 640 comprend 8 petits cubes 641 assemblés ensemble aux 8 sommets du cube 640. En réalité, les petits cubes 641 ne sont que des portions de petits cubes sur 3 faces. Sur certaines faces des petits cubes 641, sont disposées des ouvertures circulaires 643 tandis que sur d'autres faces des petits cubes 641 sont disposées des ouvertures oblongues 642. Les ouvertures circulaires 643, comme les ouvertures oblongues 642, sont destinées à recevoir les barres 611, 621 et 631 des différentes plaques 612, 622 et 632.

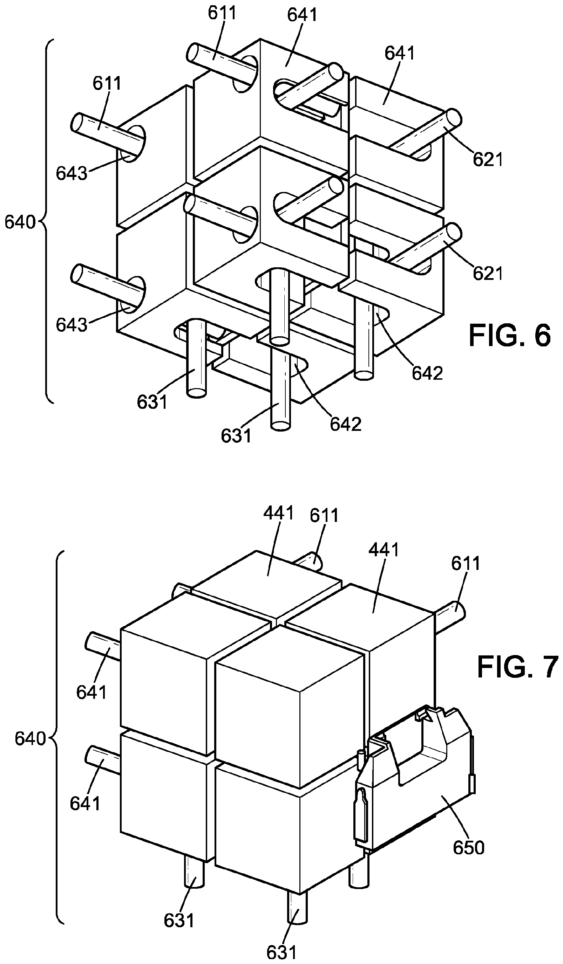

La figure 6 est une vue en perspective d'un exemple de réalisation d'assemblage entre d'une part les interfaces et d'autre part le socle d'organe d'entraînement.

Les barres 611, 621 et 631 des différentes plaques 612, 622 et 632 peuvent s'enfoncer plus ou moins dans les différentes ouvertures 642 et 643, permettant ainsi respectivement aux plaques 612, 622 et 632 de déplacer le cube 640 respectivement dans les directions Y, X et Z. Les plaques 612, 622 et 632 poussent ou tirent les petits cubes 641 formant ensemble le cube 640.

La figure 7 est une autre vue en perspective d'un exemple de réalisation d'assemblage entre d'une part les interfaces et d'autre part le socle d'organe d'entraînement.

De ce côté, les petits cubes 641 ne comportent aucune ouverture, mais seulement un embout 650 de touche. Cet embout 650 va pouvoir porter de manière solidaire et fixe l'organe d'entraînement 24 présenté au niveau de la figure 2.

## Revendications

1. Chaîne de transmission de mouvement comprenant :
- un socle (640) d'organe d'entraînement (24, 24') d'un élément mobile (15', 15"),
- trois actionneurs (610, 620, 630) pilotant le socle (640) de l'organe d'entraînement (24, 24') respectivement selon trois directions (Y, X, Z) de translation distinctes entre elles, par l'intermédiaire de trois interfaces (612, 622, 632) respectives avec le socle (640) de l'organe d'entraînement (24, 24'),
**caractérisée en ce que**
- les trois interfaces (612, 622, 632) sont sensiblement planes,
- ces trois interfaces (612, 622, 632) sont orthogonales les unes aux autres,
- et ces trois interfaces (612, 622, 632) sont imbriquées les unes dans les autres.

2. Chaîne de transmission de mouvement selon revendication 1, **caractérisée en ce que** l'intersection des surfaces moyennes des trois interfaces (612, 622, 632) est localisée en région centrale du socle (640) de l'organe d'entraînement (24, 24').

3. Chaîne de transmission de mouvement selon l'une quelconque des revendications précédentes, **caractérisée en ce que** :
- les trois directions (Y, X, Z) de translation sont orthogonales les unes aux autres.

4. Chaîne de transmission de mouvement selon l'une quelconque des revendications précédentes, **caractérisée en ce que** :
- les trois interfaces (612, 622, 632) sont des plaques (612, 622, 632) de pression transmettant les poussées respectives des trois actionneurs (610, 620, 630).

5. Chaîne de transmission de mouvement selon la revendication 4, **caractérisée en ce que** :
- la première plaque (612) comprend deux ouvertures (613, 614) orthogonales entre elles qui sont respectivement traversées par la deuxième plaque (622) et par la troisième plaque (632),
- la deuxième plaque (622) comprend une ouverture (623) qui est traversée par la troisième plaque (632), l'ouverture de la deuxième plaque (622) étant orthogonale aux deux ouvertures (613, 614) de la première plaque (612),
- la troisième plaque (632) n'est traversée ni par la première plaque (612)ni par la deuxième plaque (622).

6. Chaîne de transmission de mouvement selon la revendication 5, **caractérisée en ce que** :
- chacune des ouvertures (613, 614, 623) autorise un débattement de la plaque (622, 632, 632) qui la traverse, ce débattement correspondant à la course de l'actionneur (620, 630, 630) de la plaque (622, 632, 632) qui traverse ladite ouverture (613, 614, 623), ce débattement étant supérieur à l'épaisseur de la plaque (622, 632, 632) qui traverse ladite ouverture (613, 614, 623) .

7. Chaîne de transmission de mouvement selon l'une quelconque des revendications 4 à 6, **caractérisée en ce que** :
- chaque plaque (612, 622, 632) est mobile en translation selon une direction (Y, X, Z) parallèle à la droite constituée par l'intersection des deux autres plaques.

8. Chaîne de transmission de mouvement selon l'une quelconque des revendications 4 à 7, **caractérisée en ce que** :
- chaque plaque (612, 622, 632) est reliée à son actionneur (610, 620, 630) par deux barres (611, 621, 631) symétriques par rapport à l'axe (Y, X, Z) de poussée dudit actionneur (610, 620, 630), de préférence par quatre barres (611, 621, 631) symétriques par rapport à l'axe (Y, X, Z) de poussée dudit actionneur (610, 620, 630).

9. Chaîne de transmission de mouvement selon l'une quelconque des revendications précédentes, **caractérisée en ce que** :
- le socle (640) de l'organe d'entraînement (24, 24') est solidarisé de manière fixe à chacune des interfaces (612, 622, 632) de manière à ce que le déplacement de l'une des interfaces (612, 622, 632) entraîne automatiquement le même déplacement du socle (640) de l'organe d'entraînement (24, 24').

10. Chaîne de transmission de mouvement selon l'une quelconque des revendications précédentes, **caractérisée en ce que** :
- le socle (640) de l'organe d'entraînement (24, 24') est un cube (640) à l'intérieur duquel sont situées les trois interfaces (612, 622, 632).
- de préférence, le socle (640) de l'organe d'entraînement (24, 24') est un cube (640) résultant de l'assemblage de huit cubes (641) plus petits assemblés autour des interfaces (612, 622, 632).

11. Chaîne de transmission de mouvement selon la revendication 10 et selon l'une quelconque des revendications 4 à 8, **caractérisée en ce que** :
- chaque plaque (612, 622, 632) est coincée entre quatre cubes (641) plus petits d'un côté et quatre cubes (641) plus petits de l'autre côté.

12. Chaîne de transmission de mouvement selon l'une quelconque des revendications précédentes, **caractérisée en ce que** :
- la région centrale est le centre de gravité du socle (640) de l'organe d'entraînement.

13. Chaîne de transmission de mouvement selon l'une quelconque des revendications précédentes, **caractérisée en ce que** :
- la chaîne de transmission de mouvement comprend un élément mobile entraîné par l'organe d'entraînement (24, 24').

14. Chaîne de transmission de mouvement selon la revendication 13, **caractérisée en ce que** :
- l'élément mobile (15', 15") est un cathéter (15')ou un guide (15") de cathéter,
- l'organe d'entraînement (24, 24')est un organe de serrage (24, 24')d'un cathéter (15') ou d'un guide (15'')de cathéter.

## Patentansprüche

1. Bewegungsübertragungskette, umfassend:
- einen Sockel (640) eines Antriebselements (24, 24') eines beweglichen Elements (15', 15"),
- drei Aktuatoren (610, 620, 630), die den Sockel (640) des Antriebselements jeweils in drei voneinander verschiedenen Translationsrichtungen (Y, X, Z) über drei jeweilige Schnittstellen (612, 622, 632) mit dem Sockel (640) des Antriebselements (24, 24') steuern,
**dadurch gekennzeichnet, dass**
- die drei Schnittstellen (612, 622, 632) im Wesentlichen eben sind,
- diese Schnittstellen (612, 622 , 632 ) orthogonal zueinander sind,
- und diese drei Schnittstellen (612 , 622, 632) ineinander verschachtelt sind.

2. Bewegungsübertragungskette nach Anspruch 1, **dadurch gekennzeichnet, dass** der Schnittpunkt der Mittelflächen der drei Schnittstellen (612, 622, 632) im mittleren Bereich des Sockels (640) des Antriebselements (24, 24') lokalisiert ist.

3. Bewegungsübertragungskette nach einem der vorhergehenden Ansprüche, **dadurch gekennzeichnet, dass**:
- die drei Translationsrichtungen (Y, X, Z) orthogonal zueinander sind.

4. Bewegungsübertragungskette nach einem der vorhergehenden Ansprüche, **dadurch gekennzeichnet, dass**:
- die drei Schnittstellen (612, 622, 632) Druckplatten (612, 622, 632) sind, die die jeweiligen Schübe der drei Aktuatoren (610, 620, 630) übertragen.

5. Bewegungsübertragungskette nach Anspruch 4, **dadurch gekennzeichnet, dass**:
- die erste Platte (612) zwei zueinander orthogonale Öffnungen (613, 614) aufweist, die jeweils von der zweiten Platte (622) und der dritten Platte (632) durchquert werden,
- die zweite Platte (622) eine Öffnung (623) umfasst, die von der dritten Platte (632) durchquert wird, wobei die Öffnung der zweiten Platte (622) orthogonal zu den beiden Öffnungen (613, 614) der ersten Platte (612) ist,
- die dritte Platte (632) weder von der ersten Platte (612) noch von der zweiten Platte (622) durchquert wird.

6. Bewegungsübertragungskette nach Anspruch 5, **dadurch gekennzeichnet, dass**:
- jede der Öffnungen (613, 614, 623) einen Ausschlag der durch sie hindurchgehenden Platte (622, 632, 632) zulässt, wobei dieser Ausschlag dem Hub des Aktuators (620, 630, 630) der durch die Öffnung (613 , 614, 623) hindurchgehenden Platte (622, 632, 632) entspricht, wobei dieser Ausschlag größer ist als die Dicke der durch die Öffnung (613 , 614, 623) hindurchgehenden Platte (622, 632, 632).

7. Bewegungsübertragungskette nach einem der Ansprüche 4 bis 6, **dadurch gekennzeichnet, dass**:
- jede Platte (612, 622, 632) in einer Richtung (Y, X, Z) parallel zu der Geraden, die durch den Schnittpunkt der beiden anderen Platten gebildet wird, translatorisch beweglich ist.

8. Bewegungsübertragungskette nach einem der Ansprüche 4 bis 7, **dadurch gekennzeichnet, dass**:
- jede Platte (612, 622, 632) mit ihrem Aktuator (610, 620, 630) durch zwei Stangen (611, 621, 631) verbunden ist, die symmetrisch zur Schubachse (Y, X, Z) des Aktuators (610, 620, 630) sind, vorzugsweise durch vier Stangen (611, 621, 631), die symmetrisch zur Schubachse (Y, X, Z) des Aktuators (610, 620, 630) sind.

9. Bewegungsübertragungskette nach einem der vorhergehenden Ansprüche, **dadurch gekennzeichnet, dass**:
- der Sockel (640) des Antriebselements (24, 24') fest mit jeder der Schnittstellen (612, 622, 632) verbunden ist, so dass die Bewegung einer der Schnittstellen (612, 622, 632) automatisch die gleiche Bewegung des Sockels (640) des Antriebselements (24, 24') zur Folge hat.

10. Bewegungsübertragungskette nach einem der vorhergehenden Ansprüche, **dadurch gekennzeichnet, dass**:
- der Sockel (640) des Antriebselements (24, 24') ein Würfel (640) ist, in dessen Innerem die drei Schnittstellen (612, 622, 632) angeordnet sind,
- vorzugsweise der Sockel des Antriebselements (24, 24') ein Würfel (640) ist, der aus acht kleineren Würfel (641) zusammengesetzt ist, die um die Schnittstellen (612, 622, 632) herum zusammengesetzt sind.

11. Bewegungsübertragungskette nach Anspruch 10 gemäß einem der Ansprüche 4 bis 8, **dadurch gekennzeichnet, dass**:
- jede Lasche (612, 622, 632) zwischen vier kleineren Würfel (641) auf der einen Seite und vier kleineren Würfel (641) auf der anderen Seite eingeklemmt ist.

12. Bewegungsübertragungskette nach einem der vorhergehenden Ansprüche, **dadurch gekennzeichnet, dass**:
- der mittlere Bereich der Schwerpunkt des Sockels (640) des Antriebselements ist.

13. Bewegungsübertragungskette nach einem der vorhergehenden Ansprüche, **dadurch gekennzeichnet, dass**:
- die Kette zur Bewegungsübertragung ein bewegliches Element umfasst, das von dem Antriebselement (24, 24') angetrieben ist.

14. Bewegungsübertragungskette nach Anspruch 13, **dadurch gekennzeichnet, dass**:
- das bewegliche Element (15', 15") ein Katheter (15') oder eine Katheterführung (15") ist,
- das Antriebselement (24, 24') ein Klemmelement (24, 24') für einen Katheter (15') oder eine Katheterführung (15") ist.

## Claims

1. A chain for transmitting movement comprising:
- a base (640) of a driving member (24, 24') for a movable element (15', 15"),
- three actuators (610, 620, 630) piloting the base (640) of the driving member (24, 24') respectively along three mutually distinct translation directions (Y, X, Z), by means of three respective interfaces (612, 622, 632) with the base (640) of the driving member (24, 24'),
**characterized in that**
- the three interfaces (612, 622, 632) are substantially flat,
- these three interfaces (612, 622, 632) are mutually orthogonal,
- and these three interfaces (612, 622, 632) are nested inside each other.

2. The chain for transmitting movement according to claim 1, **characterized in that** the intersection of the average surfaces of the three interfaces (612, 622, 632) is located in the central region of the base (640) of the driving member (24, 24').

3. The chain for transmitting movement according to any one of the preceding claims, **characterized in that**:
- the three translation directions (Y, X, Z) are mutually orthogonal.

4. The chain for transmitting movement according to any one of the preceding claims, **characterized in that**:
- the three interfaces (612, 622, 632) are pressure plates (612, 622, 632) transmitting the respective thrusts from the three actuators (610, 620, 630).

5. The chain for transmitting movement according to claim 4, **characterized in that**:
- the first plate (612) comprises two mutually orthogonal openings (613, 614) which are respectively traversed by the second plate (622) and by the third plate (632),
- the second plate (622) comprises an opening (623) which is traversed by the third plate (632), the opening of the second plate (622) being orthogonal to the two openings (613, 614) of the first plate (612),
- the third plate (632) is not traversed either by the first plate (612) or by the second plate (622).

6. The chain for transmitting movement according to claim 5, **characterized in that**:
- each of the openings (613, 614, 623) allows a travel of the plate (622, 632, 632) which passes therethrough, this travel corresponding to the stroke of the actuator (620, 630, 630) of the plate (622, 632, 632) which passes through said opening (613, 614, 623), this travel being greater than the thickness of the plate (622, 632, 632) which passes through said opening (613, 614, 623).

7. The chain for transmitting movement according to any one of claims 4 to 6, **characterized in that**:
- each plate (612, 622, 632) is movable in translation along a direction (Y, X, Z) parallel to the straight-line formed by the intersection of the two other plates.

8. The chain for transmitting movement according to any one of claims 4 to 7, **characterized in that**:
- each plate (612, 622, 632) is connected to its actuator (610, 620, 630) by two struts (611, 621, 631) symmetric with respect to the thrust axis (Y, X, Z) of said actuator (610, 620, 630), preferably by four struts (611, 621, 631) symmetric with respect to the thrust axis (Y, X, Z) of said actuator (610, 620, 630).

9. The chain for transmitting movement according to any one of the preceding claims, **characterized in that**:
- the base (640) of the driving member (24, 24') is fixedly secured with each of the interfaces (612, 622, 632) so that the movement of one of the interfaces (612, 622, 632) automatically leads to the same movement of the base (640) of the driving member (24, 24').

10. The chain for transmitting movement according to any one of the preceding claims, **characterized in that**:
- the base (640) of the driving member (24, 24') is a cube (640) inside which the three interfaces (612, 622, 632) are located,
- preferably, the base (640) of the driving member (24, 24') is a cube (640) resulting from the assembly of eight smaller cubes (641) assembled around interfaces (612, 622, 632).

11. The chain for transmitting movement according to claim 10 and according to any one of claims 4 to 8, **characterized in that**:
- each plate (612, 622, 632) is lodged between four smaller cubes (641) on one side and four smaller cubes (641) on the other side.

12. The chain for transmitting movement according to any one of the preceding claims, **characterized in that**:
- the central region is the center of gravity of the base (640) of the driving member.

13. The chain for transmitting movement according to any one of the preceding claims, **characterized in that**:
- the chain for transmitting movement comprises a movable element driven by the driving member (24, 24').

14. The chain for transmitting movement according to claim 13, **characterized in that**:
- the movable element (15', 15") is a catheter (15') or a catheter guide (15"),
- the driving member (24, 24') is a tightening member (24, 24') for a catheter (15') or a catheter guide (15").
